(19) Europäisches Patentamt
European Patent Office
Office européen des brevets

(11) Veröffentlichungsnummer: **0 048 377**
**B1**

(12) # EUROPÄISCHE PATENTSCHRIFT

(45) Veröffentlichungstag der Patentschrift: **19.10.83**

(51) Int. Cl.³: **C 07 C 125/067**, C 07 D 307/52, C 07 D 309/04

(21) Anmeldenummer: **81107018.4**

(22) Anmeldetag: **07.09.81**

(54) **Verfahren zur Herstellung von N-substituierten Imido-dicarbonsäure-diarylestern.**

(30) Priorität: **19.09.80 DE 3035393**

(43) Veröffentlichungstag der Anmeldung: **31.03.82 Patentblatt 82/13**

(45) Bekanntmachung des Hinweises auf die Patenterteilung: **19.10.83 Patentblatt 83/42**

(84) Benannte Vertragsstaaten: **BE CH DE FR GB IT LI NL**

(56) Entgegenhaltungen:
**GB-A-484 683**
**HOUBEN-WEYL »Methoden der organischen Chemie«, 1952, 4. Auflage, Band VIII: »Sauerstoffverbindungen III« GEORG THIEME VERLAG, Stuttgart**

(73) Patentinhaber: **BAYER AG, Zentralbereich Patente, Marken und Lizenzen, D-5090 Leverkusen 1, Bayerwerk (DE)**

(72) Erfinder: **Dickoré, Karlfried, Dr., Nicolai-Hartmann-Strasse 19, D-5090 Leverkusen (DE)**
Erfinder: **Kühle, Engelbert, Dr., von Bodelschwingh-Strasse 42, D-5060 Berg.-Gladbach 2 (DE)**

**EP 0 048 377 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Herstellung von N-substituierten Imidodicarbonsäure-diarylestern

Die Erfindung betrifft ein neues, einstufiges Verfahren zur Herstellung von N-substituierten Imido-dicarbonsäurediarylestern, welche als Zwischenprodukte für die Synthese von herbiziden Wirkstoffen verwendet werden können.

Es wurde gefunden, daß man N-substituierte Imido-dicarbonsäure-diarylester der allgemeinen Formel I

$$R^1-N(CO-OR^2)_2 \qquad (I)$$

in welcher

$R^1$ für einen gegebenenfalls substituierten aliphatischen, cycloaliphatischen, araliphatischen, aromatischen oder heterocyclischen Rest steht, und
$R^2$ für einen gegebenenfalls substituierten Arylrest steht,

erhält, wenn man Aminsalze der allgemeinen Formel II

$$R^1-NH_2 \cdot HX^1 \qquad (II)$$

in welcher

$R^1$ die oben angegebene Bedeutung hat und
$X^1$ für Halogen steht,

mit Kohlensäure-arylester-halogeniden der allgemeinen Formel III

$$R^2O-CO-X^2 \qquad (III)$$

in welcher

$R^2$ die oben angegebene Bedeutung hat und
$X^2$ für Halogen steht,

ohne Zusatz eines Säurebindemittels bei Temperaturen zwischen 100 und 300°C umsetzt.

Es ist als überraschend anzusehen, daß die erfindungsgemäße Umsetzung abläuft, da Aminsalze bei niedrigen Temperaturen nicht mit Kohlensäureesterhalogeniden reagieren und die freien Amine in Gegenwart eines säurebindenden Mittels nur die N-substituierten Carbamidsäurearylester liefern. Außerdem war zu erwarten, daß bei hoher Temperatur eine Rückspaltung der in erster Stufe gebildeten N-substituierten Carbamidsäurearylester erfolgen würde (vgl. Houben-Weyl, Methoden der org. Chemie, 4. Aufl., Bd. 8, S. 127 (1952)).

Verwendet man Neopentylamin-hydrochlorid und Kohlensäurephenylester-chlorid als Ausgangsstoffe so kann der Reaktionsverlauf durch das folgende Reaktionsschema wiedergegeben werden:

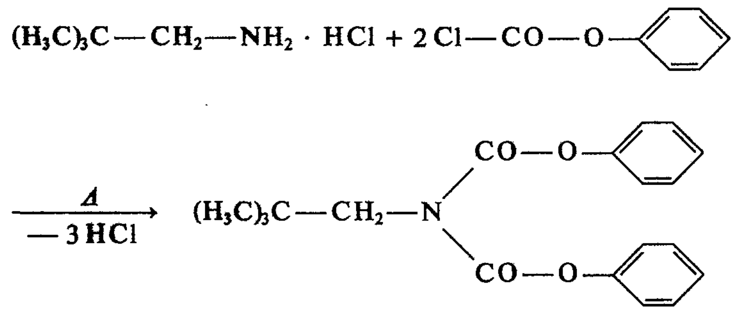


Die als Ausgangsstoffe zu verwendenden Aminsalze sind durch die Formel (II) allgemein definiert. In dieser Formel steht $R^1$ vorzugsweise für einen geradkettigen oder verzweigten Alkylrest mit 1—10 C-Atomen, der gegebenenfalls durch Niederalkoxy, Niederalkylmercapto, Halogen (insbesondere Chlor und Fluor), Cyano oder Nitro substituiert sein kann; einen Alkenylrest mit 3—8 C-Atomen; einen Alkinylrest mit 3—8 C-Atomen; einen cycloaliphatischen Rest mit 3—8 Ring-C-Atomen, der gegebenenfalls durch Niederalkyl substituiert sein kann; einen araliphatischen Rest mit insgesamt 7—12 C-Atomen, wobei das aromatische Ringsystem gegebenenfalls durch Halogen, Nitro, Trifluormethyl, Cyano, Niederalkyl und/oder Niederalkoxy substituiert sein kann; einen aromatischen Rest mit 6—12 C-Atomen, der gegebenenfalls durch Halogen, Nitro, Trifluormethyl, Cyano,

Niederalkyl und/oder Niederalkoxy substituiert sein kann, oder für einen heterocyclischen Rest mit 5—6 Ringatomen, wobei 1—3 Heteroatome, wie Sauerstoff, Schwefel und/oder Stickstoff im Ringsystem vorhanden sein können. Die Ausdrücke »Niederalkyl«, »Niederalkoxy« bzw. »Niederalkylmercapto« sollen im Rahmen dieser Erfindung entsprechende Reste mit jeweils 1—4 C-Atomen bedeuten.

$X^1$ steht in dieser Formel vorzugsweise für Fluor, Chlor oder Brom, insbesondere für Chlor.

Die erfindungsgemäß verwendbaren primären Amine, die den Aminsalzen (II) zugrunde liegen, sind alle aus der Literatur bekannt (vgl. z. B. Houben-Weyl, Methoden der Organischen Chemie, 4. Aufl., B. XI/1, S. 9—1033 (1957). Die halogenwasserstoffsauren Salze können nach bekannten Verfahren aus den Aminen durch Addition des entsprechenden Halogenwasserstoffes hergestellt werden.

Als Beispiele für die den Hydrohalogeniden der Formel (II) zugrunde liegenden primären Amine seien im einzelnen genannt:

Methylamin
Ethylamin
Propylamin
Isopropylamin
1,1-Dimethyl-propylamin
1,2-Dimethyl-propylamin
2,2-Dimethyl-propylamin (Neopentylamin)
1,2,2-Trimethyl-propylamin
1-Ethyl-propylamin
1-Isopropyl-2-methyl-propylamin
Butylamin
Isobutylamin
sek.-Butylamin
tert.-Butylamin
1-Methyl-butylamin
2,2-Dimethyl-butylamin
3,3-Dimethyl-butylamin
1,3,3-Trimethyl-butylamin
Pentylamin
1-Methyl-pentylamin
2,2-Dimethyl-pentylamin
1,2,2-Trimethyl-pentylamin
Hexylamin
2-Ethyl-hexylamin
1-Methyl-oktylamin
Allylamin
2-Methyl-allylamin
Propargylamin
Cyclopropylamin
Cyclopropyl-methylamin
Cyclobutylamin
Cyclopentylamin
Cyclopentyl-methylamin
Cyclohexylamin
Cyclohexyl-methylamin
3-Methyl-cyclohexylamin
4-Methyl-cyclohexylamin
4-tert.-Butyl-cyclohexylamin
4-Methyl-cyclohexyl-methylamin
3,3,5-Trimethyl-cyclohexylamin
Cyclohexen(3)-yl-methylamin
3,4-Dimethyl-cyclohexen(3)-ylamin
Cyclohexen(3)-yl-methylamin
3,4-Dimethyl-cyclohexen(3)-yl-methylamin
Cycloheptanylamin
Cycloheptanyl-methylamin
Cyclooktanylamin
Cyclooktanyl-methylamin
Cyclododekanylamin
Cyclododekanyl-methylamin
Adamantyl-methylamin
2-(Bicyclo[2,2,1]heptyl)-methylamin

6-(2,3,3a,4,5,6,7,7a-Oktahydro-4,7-methanoindenyl)-methylamin
2-(1,2,3,4,5,6,7,8,8a,4a-Dekahydro-1,4 : 5,8-dimethanonaphthyl)-methylamin
5-(4,5,6,7,7a,3a-Hexahydro-indenyl)-methylamin
2-Chlor-ethylamin
2,2,2-Trifluor-ethylamin
3,3-Dichlor-3-fluor-propylamin
3,3,3-Trifluor-propylamin
2,2-Difluor-propylamin
2,2,2-Trifluor-1-methyl-ethylamin
6-Chlor-hexylamin
3-Trifluormethyl-cyclohexylamin
4-Trifluormethyl-cyclohexylamin
3-Trifluormethyl-cyclohexyl-methylamin
4-Trifluormethyl-cyclohexyl-methylamin
2-Methoxy-ethylamin
3-Methoxy-propylamin
5-Cyano-pentylamin
2-Ethoxycarbonyl-ethylamin
Anilin
2-Chloranilin
3-Chloranilin
4-Chloranilin
2,4-Dichloranilin
3,4-Dichloranilin
3,5-Dichloranilin
2,4,5-Trichloranilin
3-Nitroanilin
4-Nitroanilin
3-Chlor-4-nitroanilin
2-Methylanilin
2-Chlor-6-methylanilin
4-Chlor-2-trifluormethylanilin
3-Methylanilin
3-Trifluormethylanilin
4-Methylanilin
2,6-Dimethylanilin
2-Ethylanilin
2-Ethyl-6-methylanilin
2,6-Diethylanilin
Benzylamin
1-Phenyl-ethylamin
2-Phenyl-ethylamin
2-Chlor-benzylamin
2,4-Dichlor-benzylamin
Furyl(2)-methylamin
Tetrahydro-furyl(2)-methylamin
Tetrahydro-pyranyl(2)-methylamin
Tetrahydro-pyranyl(3)-methylamin.

Die weiterhin als Ausgangsstoffe zu verwendenden Kohlensäure-arylester-halogenide sind durch die Formel (II) allgemein definiert. In dieser Formel steht $R^2$ vorzugsweise für einen gegebenenfalls durch Chlor, Methyl und/oder Methoxy substituierten Phenyl- oder Naphthylrest.

$X^2$ in Formel (II) steht vorzugsweise für Chlor oder Fluor, wobei die Reste $X^1$ und $X^2$ gleich oder verschieden sein können.

Die erfindungsgemäß verwendbaren Kohlensäure-arylester-halogenide der Formel (III) sind bekannt oder können nach bekannten Verfahren hergestellt werden. So kann man z. B. die Kohlensäure-phenylester-chloride in an sich bekannter Weise durch Phosgenierung von Phenolen herstellen (vgl. Houben-Weyl, Methoden der organischen Chemie, 4. Aufl., Bd. 8, S. 103 (1952)); die entsprechenden Kohlensäure-phenylester-fluoride können analog aus Phenolen und Difluorphosgen gewonnen werden (vgl. J. Chem. Soc. [London] 1948, S. 2183).

Als Beispiele für Ausgangsverbindungen der Formel (III) seien im einzelnen genannt:
die Kohlensäureester-chloride des Phenols, 4-Chlorphenols, 4-Kresols oder 1-Naphthols sowie das Kohlensäureester-fluorid des Phenols.

Die erfindungsgemäße Umsetzung kann in Abwesenheit oder in Anwesenheit eines Verdünnungsmittels durchgeführt werden. Arbeitet man ohne Verdünnungsmittel, so wird das Amin-hydrohalogenid (II) am zweckmäßigsten portionsweise in das im Reaktionsgefäß befindliche, auf die Reaktionstemperatur erwärmte Kohlensäure-arylester-halogenid (III) eingetragen bzw. mit einer Schneckenvorrichtung gleichmäßig eindosiert.

Als Verdünnungsmittel für das Kohlensäure-arylesterhalogenid (III), das im allgemeinen vorgelegt wird, kommen hochsiedende inerte organische Lösungsmittel, wie chlorierte oder nitrierte aromatische Kohlenwasserstoffe, z. B. Chlorbenzol, die Dichlorbenzole, die Trichlorbenzole oder Nitrobenzole, in Frage.

Außerdem ist es möglich und in vielen Fällen besonders vorteilhaft, die Reaktion in einem Überschuß des als Reaktionskomponente dienenden Kohlensäure-arylester-halogenids (III) durchzuführen.

Führt man die erfindungsgemäße Umsetzung in Gegenwart eines inerten organischen Lösungsmittels als Verdünnungsmittel durch, so setzt man auf 1 Mol eines Aminsalzes der Formel (II) im allgemeinen 2—15 Mol, vorzugsweise 3—12 Mol eines Kohlensäure-arylester-halogenids der Formel (III) ein. Verwendet man dagegen als Verdünnungsmittel einen Überschuß an Kohlensäure-halogenid (III), so kann man auf 1 Mol Aminsalz der Formel (II) bis zu 20 Mol, zweckmäßigerweise jedoch etwa 4—15 Mol, bevorzugt etwa 8—12 Mol Kohlensäure-arylester-halogenid (III) einsetzen. Es empfiehlt sich also in jedem Falle, das Kohlensäure-arylester-halogenid (III) in überstöchiometrischen Mengen einzusetzen.

Das erfindungsgemäße Verfahren wird ohne Zusatz eines Säurebindemittels durchgeführt. Es hat sich jedoch als vorteilhaft erwiesen, den im Verlauf der Reaktion gebildeten Halogenwasserstoff rasch aus dem Reaktionsgemisch zu entfernen. Dies wird am zweckmäßigsten dadurch erreicht, daß man einen ständigen Luft- oder Stickstoffstrom durch das Reaktionsgemisch leitet (vgl. Herstellungsbeispiele).

Die Reaktionstemperaturen liegen, wie oben angegeben, zwischen 100 und 300° C, vorzugsweise zwischen 170 und 250° C.

Die erfindungsgemäße Reaktion wird im allgemeinen bei Normaldruck durchgeführt.

Die Isolierung der Reaktionsprodukte erfolgt in einfacher Weise durch destillative Trennung des Reaktionsgemisches. Feste, höher schmelzende Imido-dicarbonsäure-diarylester lassen sich auch leicht durch Umkristallisieren reinigen.

Die erfindungsgemäß herstellbaren N-substituierten Imidodicarbonsäure-diarylester (I) können als Zwischenprodukte zur Herstellung bekannter herbizider Wirkstoffe aus der Reihe der 1,3,5-Triazin-2,4(1H,3H)-dione verwendet werden (vgl. z. B. DE-OS 2 254 200 und US-PS 4 056 527 sowie EP-A2-0 034 750, EP-A2-0 034 751, EP-A1-0 048 376 und EP-A1-0 048 377).

Die nachfolgenden Herstellungsbeispiele sollen zur weiteren Erläuterung der Erfindung dienen.

## Herstellungsbeispiele

### Beispiel 1

$$(CH_3)_3C-CH_2-N(CO-O-C_6H_5)_2 \qquad (1)$$

In einem 2-Liter-Vierhalskolben werden 810 ml (6,4 Mol) Kohlensäure-phenylester-chlorid zum Sieden erhitzt und unter Rühren und Durchleiten von Stickstoff werden mittels einer Schneckenvorrichtung 123,5 g (1 Mol) Neopentylamin-hydrochlorid im Verlaufe von 2—3 Stunden eindosiert. Anschließend wird noch 6 Stunden bei Siedehitze nachgerührt.

Eine gaschromatographische Analyse dieser Reaktionslösung liefert — ohne Berücksichtigung des im Überschuß eingesetzten Kohlensäure-phenylester chlorids — folgende Werte:

10,7% — Neopentylisocyanat
20,1% — Diphenylcarbonat
63,3% — N-Neopentyl-imido-dicarbonsäure-diphenylester (1)
4,0% — Orthokohlensäure-tetraphenylester

Die erhaltene Reaktionslösung wird destilliert aufgearbeitet.

Nach Abdestillieren des überschüssigen Kohlensäure-phenylester-chlorids im Wasserstrahlvakuum (Siedepunkt 75° C bei 16 mbar) wird das Diphenylcarbonat im Hochvakuum bei einer Badtemperatur von 140—150° C abdestilliert.

Der verbleibende Rückstand besteht aus 96%igem N-Neopentyl-imido-dicarbonsäure-diphenylester. Ausbeute: 238 g (70% der Theorie). Eine aus Petrolether umkristallisierte Probe schmilzt bei 81° C. Der Siedepunkt beträgt 156° C bei 0,02 mbar.

Das als Ausgangsprodukt benötigte Neopentylamino-hydrochlorid kann wie folgt hergestellt werden:

In eine Lösung von 87 g (1 Mol) Neopentylamin in 500 ml Toluol leitet man unter Rühren 40 g Chlorwasserstoffgas ein, saugt ab und trocknet bei 150° C im Trockenschrank. Man erhält 120 g (97%

der Theorie) Neopentylamin-hydrochlorid in Form eines feinen Pulvers, Schmelzpunkt 300° C.

In analoger Weise könnnen die in der folgenden Tabelle aufgeführten Verbindungen der allgemeinen Formel (I) hergestellt werden:

**Tabelle**

$R^1—N(CO—OR^2)_2$ (I)

| Beispiel Nr. | $R^1$ | $R^2$ | Schmelzpunkt (°C) | Siedepunkt (°C/mbar) |
|---|---|---|---|---|
| 2 | $CH_3—$ | $—C_6H_5$ | 102 – 105 | |
| 3 | $(CH_3)_2CH—$ | $—C_6H_5$ | 35 – 37 | 155/0,07 |
| 4 | $C_2H_5—CH—$<br>\|<br>$CH_3$ | $—C_6H_5$ | | 165 – 170/0,2 |
| 5 | $(CH_3)_2CH—CH_2—$ | $—C_6H_5$ | 40 | 160/0,1 |
| 6 | $(CH_3)_3C—$ | $—C_6H_5$ | 132 | 150/0,1 |
| 7 | $(CH_3)_3C—CH_2—$ | 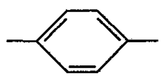 $CH_3$ | 71 – 73 | 172/0,008 |
| 8 | $(CH_3)_3C—CH_2—$ | 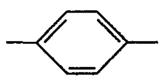 Cl | 72 – 73 | 182/0,03 |
| 9 | $(CH_3)_3C—CH_2—$ | 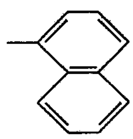 | 92 – 93 | |
| 10 | $CH_3$<br>\|<br>$C_2H_5—C—$<br>\|<br>$CH_3$ | $—C_6H_5$ | 95 – 97 | 150/0,05 |
| 11 | $(CH_3)_3C—CH—$<br>\|<br>$CH_3$ | $—C_6H_5$ | 40 – 42 | 159/0,15 |
| 12 | $CH_3$<br>\|<br>$C_2H_5—C—CH_2—$<br>\|<br>$CH_3$ | $—C_6H_5$ | 49 – 50 | 178/0,05 |
| 13 | $[(CH_3)_2CH—]_2CH—$ | $—C_6H_5$ | | 158 – 161/0,02 |
| 14 | $C_4H_9—CH—CH_2$<br>\|<br>$C_2H_5$ | $—C_6H_5$ | | 182 – 185/0,06 |
| 15 | $C_7H_{15}—CH—$<br>\|<br>$CH_3$ | $—C_6H_5$ | | 173/0,09 |

Fortsetzung

| Beispiel Nr. | $R^1$ | $R^2$ | Schmelzpunkt (°C) | Siedepunkt (°C/mbar) |
|---|---|---|---|---|
| 16 | $(C_2H_5)_2CH-$ | $-C_6H_5$ | 54 – 56 | 140/0,12 |
| 17 | $CH_2{=}CH-CH_2-$ | $-C_6H_5$ | | 153/0,04 |
| 18 | 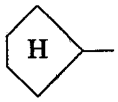 | $-C_6H_5$ | 53 | 160/0,1 |
| 19 | 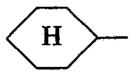 | $-C_6H_5$ | 85 | 175/0,09 |
| 20 | $(CH_3)_3C-$ 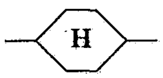 | $-C_6H_5$ | | 186 – 200/0,001 |
| 21 | $H_3C-$ 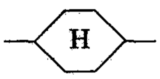 $-CH_2-$ | $-C_6H_5$ | | 185 – 188/0,06 |
| 22 | 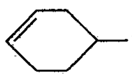 $-CH_2-$ | $-C_6H_5$ | | 195 – 200/0,09 |
| 23 | $CH_3$ $H_3C-$ 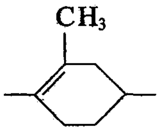 $-CH_2-$ | $-C_6H_5$ | | 200 – 205/0,07 |
| 24 | 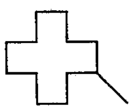 | $-C_6H_5$ | 57 – 62 | 210/0,6 |
| 25 | 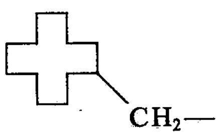 $CH_2-$ | $-C_6H_5$ | | 240 – 250/0,1 |
| 26 | 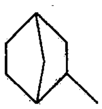 $CH_2-$ | $-C_6H_5$ | 84 – 86 | 202/0,02 |
| 27 | 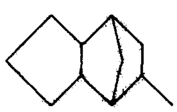 $CH_2-$ | $-C_6H_5$ | 92 – 94 | |
| 28 | 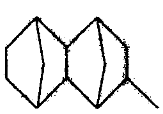 $CH_2-$ | $-C_6H_5$ | | 232 – 240/0,07 |
| 29 | 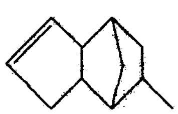 $CH_2-$ | $-C_6H_5$ | | 251 – 220/0,07 |

Fortsetzung

| Beispiel Nr. | $R^1$ | $R^2$ | Schmelzpunkt (°C) | Siedepunkt (°C/mbar) |
|---|---|---|---|---|
| 30 | $Cl(CH_2)_2—$ | $—C_6H_5$ | | 177 – 190/0,1 |
| 31 | $F_3C—CH_2—$ | $—C_6H_5$ | 76 | 140/0,3 |
| 32 | $Cl_2FC—(CH_2)_2—$ | $—C_6H_5$ | 68 – 70 | 187/0,008 |
| 33 | $F_3C—(CH_2)_2—$ | $—C_6H_5$ | 66 – 69 | 147 – 153/0,3 |
| 34 | $CH_3—CF_2—CH_2—$ | $—C_6H_5$ | 60 – 62 | 160 – 164/0,08 |
| 35 | 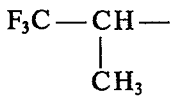 | $—C_6H_5$ | | 150/0,17 |
| 36 | $Cl—(CH_2)_6—$ | $—C_6H_5$ | | 210/0,1 |
| 37 | $H_3C—O—(CH_2)_2—$ | $—C_6H_5$ | | 168/0,1 |
| 38 | $H_3C—O—(CH_2)_3—$ | $—C_6H_5$ | | 168 – 170/0,08 |
| 39 | $NC—(CH_2)_5—$ | $—C_6H_5$ | | 230 – 235/0,1 |
| 40 | $C_2H_5-O-CO-(CH_2)_2-$ | $—C_6H_5$ | | 190 – 193/0,08 |
| 41 | $C_6H_5—$ | $—C_6H_5$ | 124 – 125 | |
| 42 | 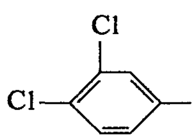 | $—C_6H_5$ | 97 – 100 | |
| 43 | 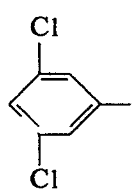 | $—C_6H_5$ | 178 – 180 | |
| 44 | $C_6H_5—CH_2—$ | $—C_6H_5$ | 70 – 72 | 191/0,01 |
| 45 | 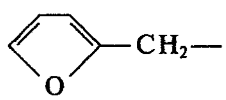 | $—C_6H_5$ | | 190 – 200/0,1 |
| 46 | 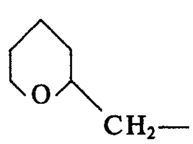 | $—C_6H_5$ | 78 – 79 | 176/0,007 |

**Patentansprüche**

1. Verfahren zur Herstellung von N-substituierten Imido-dicarbonsäure-diarylestern der allgemeinen Formel I

$$R^1-N(CO-OR^2)_2 \quad (I)$$

in welcher

$R^1$ für einen gegebenenfalls substituierten aliphatischen, cycloaliphatischen, araliphatischen, aromatischen oder heterocyclischen Rest steht, und
$R^2$ für einen gegebenenfalls substituierten Arylrest steht,

dadurch gekennzeichnet, daß man Aminsalze der allgemeinen Formel II

$$R^1-NH_2 \cdot HX^1 \qquad (II)$$

in welcher

$R^1$ die oben angegebene Bedeutung hat und
$X^1$ für Halogen steht,

mit Kohlensäure-arylester-halogeniden der allgemeinen Formel III

$$R^2O-CO-X^2 \qquad (III)$$

in welcher

$R^2$ die oben angegebene Bedeutung hat und
$X^2$ für Halogen steht,

ohne Zusatz eines Säurebindemittels bei Temperaturen zwischen 100 und 300° C umsetzt.

2. Verfahren nach Anspruch 1, dadurch gekennzeichnet, daß man die Umsetzung bei Temperaturen zwischen 170 und 250° C durchführt.

3. Verfahren nach Anspruch 1, dadurch gekennzeichnet, daß man das Kohlensäure-arylester-halogenid (III) in überstöchiometrischen Mengen einsetzt.

4. Verfahren nach Anspruch 1, dadurch gekennzeichnet, daß man Aminsalze der allgemeinen Formel (II) einsetzt, in welcher

$R^1$ für einen geradkettigen oder verzweigten Alkylrest mit 1—10 C-Atomen, der gegebenenfalls durch Niederalkoxy, Niederalkylmercapto, Halogen, Cyano oder Nitro substituiert sein kann; einen Alkenylrest mit 3—8 C-Atomen; einen Alkinylrest mit 3—8 C-Atomen; einen cycloaliphatischen Rest mit 3—8 C-Atomen, der gegebenenfalls durch Niederalkyl substituiert sein kann; einen araliphatischen Rest mit 7—12 C-Atomen , wobei das aromatische Ringsystem gegebenenfalls durch Halogen, Nitro, Trifluormethyl, Cyano, Niederalkyl und/oder Niederalkoxy substituiert sein kann; einen aromatischen Rest mit 6—12 C-Atomen, der gegebenenfalls durch Halogen, Nitro, Trifluormethyl, Cyano, Niederalkyl und/oder Niederalkoxy substituiert sein kann, oder für einen heterocyclischen Rest mit 5—6 Ringatomen, wobei 1—3 Heteroatome im Ringsystem vorhanden sein können, steht, und
$X^1$ für Chlor steht.

5. Verfahren nach Anspruch 1, dadurch gekennzeichnet, daß man Kohlensäure-arylester-halogenide der Formel (III) einsetzt, in welcher

$R^2$ für einen gegebenenfalls durch Chlor, Methyl und/oder Methoxy substituierten Phenyl- oder Naphthylrest steht und
$X^2$ für Chlor oder Fluor steht.

6. Verfahren nach Anspruch 1, dadurch gekennzeichnet, daß man Neopentylamin-hydrochlorid mit Kohlensäure-phenylester-chlorid umsetzt.

**Claims**

1. Process for the production of N-substituted imidodicarboxylic acid diaryl esters of the general formula I

$$R^1-N(CO-OR^2)_2 \qquad (I)$$

in which

$R^1$ represents an optionally substituted aliphatic, cycloaliphatic, araliphatic, aromatic or heterocyclic

radical and

$R^2$ represents an optionally substituted aryl radical,

characterised in that amine salts of the general formula II

$$R^1-NH_2 \cdot HX^1 \qquad (II)$$

in which

$R^1$ has the meaning indicated above and

$X^1$ represents halogen,

are reacted with carbonic acid aryl ester halides of the general formula III

$$R^2O-CO-X^2 \qquad (III)$$

in which

$R^2$ has the meaning indicated above and

$X^2$ represents halogen,

at temperatures between 100 and 300° C without the addition of an acid-binding agent.

2. Process according to Claim 1, characterised in that the reaction is carried out at temperatures between 170 and 250° C.

3. Process according to Claim 1, characterised in that the carbonic acid aryl ester halide (III) is employed in amounts which are greater than the stoichiometric amount.

4. Process according to Claim 1, characterised in that amine salts of the general formula (II) in which

$R^1$ represents a straight-chain or branched alkyl radical which has 1—10 C atoms and can optionally be substituted by lower alkoxy, lower alkylmercapto, halogen, cyano or nitro; an alkenyl radical with 3—8 C atoms; an alkinyl radical with 3—8 C atoms; a cycloaliphatic radical which has 3—8 C atoms and can optionally be substituted by lower alkyl; an araliphatic radical with 7—12 C atoms, it being possible for the aromatic ring system to be optionally substituted by halogen nitro, trifluoromethyl, cyano, lower alkyl and/or lower alkoxy; an aromatic radical which has 6—12 C atoms and can optionally be substituted by halogen, nitro, trifluoromethyl, cyano, lower alkyl and/or lower alkoxy, or a heterocyclic radical with 5—6 ring atoms, it being possible for 1—3 hetero atoms to be present in the ring system, and

$X^1$ represents chlorine,

are employed.

5. Process according to Claim 1, characterised in that carbonic acid aryl ester halides of the formula (III) in which

$R^2$ represents a phenyl or naphthyl radical which is optionally substituted by chlorine, methyl and/or methoxy and

$X^2$ represents chlorine or fluorine.

6. Process according to Claim 1, characterised in that neopentylamino hydrochloride is reacted with carbonic acid phenyl ester chloride.

## Revendications

1. Procédé de production d'esters diaryliques d'acides imidodicarboxyliques N-substitués de formule générale I

$$R^1-N(CO-OR^2)_2 \qquad (I)$$

dans laquelle

$R^1$ est un reste aliphatique, cycloaliphatique, araliphatique, aromatique ou hétérocyclique éventuellement substitué, et

$R^2$ est un reste aryle éventuellement substitué, caractérisé en ce qu'on fait réagir à des températures comprises entre 100 et 300° C, sans addition d'accepteur d'acide, des sels d'amines de formule générale II:

$$R^1-NH_2 \cdot HX^1 \quad (II)$$

dans laquelle

$R^1$ a la définition indiquée ci-dessus et
$X^1$ est un halogène,

avec des halogénures d'esters aryliques d'acide carbonique de formule générale III

$$R^2O-CO-X^2 \quad (III)$$

dans laquelle

$R^2$ a la définition indiquée ci-dessus et
$X^2$ est un halogène.

2. Procédé suivant la revendication 1, caractérisé en ce qu'on conduit la réaction à des températures comprises entre 170 et 250° C.
3. Procédé suivant la revendication 1, caractérisé en ce qu'on utilise l'halogénure d'ester arylique d'acide carbonique (III) en quantités surstoechiométriques.
4. Procédé suivant la revendication 1, caractérisé en ce qu'on utilise des sels d'amines de formule générale (II), dans laquelle

$R^1$ désigne un reste alkyle à chaîne droite ou ramifié ayant 1 à 10 atomes de carbone, qui peut être substitué, le cas échéant, par un radical alkoxy inférieur, alkylmercapto inférieur, halogéno, cyano ou nitro; un reste alcényle ayant 3 à 8 atomes de carbone; un groupe alcynyle ayant 3 à 8 atomes de carbone; un reste cycloaliphatique ayant 3 à 8 atomes de carbone, qui peut être substitué, le cas échéant par un radical alkyle inférieur; un reste araliphatique ayant 7 à 12 atomes de carbone, le noyau aromatique pouvant alors être substitué, le cas échéant, par un radical halogéno, nitro, trifluorométhyle, cyano, alkyle inférieur et/ou alkoxy inférieur; un reste aromatique ayant 6 à 12 atomes de carbone, qui peut être substitué, le cas échéant par un radical halogéno, nitro, trifluorométhyle, cyano, alkyle inférieur et/ou alkoxy inférieur, ou un reste hétérocyclique à noyau de 5 ou 6 atomes, 1 à 3 hétéro-atomes pouvant êtres présents dans le noyau, et
$X^1$ désigne le chlore.

5. Procédé suivant la revendication 1, caractérisé en ce qu'on utilise des halogénures d'esters aryliques d'acide carbonique de formule (III), dans lesquels

$R^2$ désigne un reste phényle ou naphtyle éventuellement substitué par un radical chloro, méthyle et/ou méthoxy et
$X^2$ désigne du chlore ou du fluor.

6. Procédé suivant la revendication 1, caractérisé en ce qu'on fait réagir le chlorhydrate de néopentylamine avec le chlorure de l'ester phénylique de l'acide carbonique.